# EUROPÄISCHE PATENTSCHRIFT

(11) **EP 1 917 064 B1**
(45) Veröffentlichungstag und Bekanntmachung des Hinweises auf die Patenterteilung: **04.06.2014**
(21) Anmeldenummer: 06704435.4
(22) Anmeldetag: 11.01.2006
(51) Int. Cl.: A61N 1/06

(54) **MEDIZINISCHES ELEKTRODENSYSTEM**
MEDICAL ELECTRODE SYSTEM
SYSTEME D'ELECTRODE MEDICAL

(30) Priorität: 13.01.2005 DE 202005000544 U
(43) Veröffentlichungstag der Anmeldung: 07.05.2008
(73) Patentinhaber: Mantsch, Christian, 32427 Minden (DE); Stumpp, Uwe, 78665 Frittlingen (DE)
(72) Erfinder: Mantsch, Christian, 32427 Minden (DE); Stumpp, Uwe, 78665 Frittlingen (DE)
(74) Vertreter: Wiebusch, Manfred
(86) Internationale Anmeldenummer: PCT/EP2006/000193
(87) Internationale Veröffentlichungsnummer: WO 2006/074917

(56) Entgegenhaltungen:
- US-A- 5 121 754
- US-A1- 2002 198 568
- US-A1- 2003 135 253

## Beschreibung

Die Erfindung betrifft ein Elektrodensystem mit einer implantierbaren, flexiblen Elektrode, insbesondere Epiduralelektrode, mit mindestens einem distalen elektrischen Kontakt. Gemäß einer Weiterentwicklung ist die Elektrode in einem implantierbaren, flexiblen Katheter, insbesondere Epiduralkatheter, angeordnet.

Ein solcher Katheter hat üblicherweise mindestens einen Kanal, der beispielsweise zur Verabreichung von Medikamenten durch den Katheter dient. Mitunter wird aber auch eine Elektrode ohne einen Kanal als Katheter bezeichnet, beispielsweise als Stimulationskatheter. Solche Katheter mit einem distalen elektrischen Kontakt, die keinen Kanal aufweisen und somit keine Katheter im eigentlichen Sinne sind, werden im folgenden als Elektroden bezeichnet.

Katheter sind bekannte medizintechnische Produkte, die für verschiedene Einsatzzwecke in der Diagnostik oder Therapie hergestellt werden. So sind beispielsweise Epiduralkatheter bekannt, die von einem Arzt in den Periduralspalt im Bereich des Wirbelkanals eingeführt werden können, um beispielsweise schmerzstillende Medikamente injizieren zu können. Ein solches Verfahren wird insbesondere zur Behandlung chronischer Schmerzen angewandt. Dabei kann der Katheter beispielsweise eine Zeit von 1 bis 30 Tagen im Körper verbleiben, und die Injektion der Medikamente kann über externe oder implantierte Pumpen erfolgen.

Anstelle von Kathetern werden auch Elektroden in der Therapie chronischer Schmerzen eingesetzt. So sind Elektroden zur Implantierung bekannt, die zur Dauerstimulation des Rückenmarkes oder der Nerven an einen Impulsgenerator angeschlossen werden.

Ferner sind Spezialnadeln bekannt, die an einen Generator für gepulste Hochfrequenz angeschlossen werden. Solche Spezialnadeln und Hochfrequenzgeneratoren werden verwendet, um durch gezielte Reizung von Nerven die Freisetzung von schmerzhemmenden Substanzen im Rückenmark auszulösen und dadurch eine Schmerzbehandlung zu bewirken. Dieser Einsatz dieser Spezialnadeln stößt jedoch häufig an anatomische Grenzen oder wird wegen der Gefahr der Verletzung beim Einführen der Spezialnadeln vermieden.

Aus US 2002/198568 A1 ist ein System mit einer Metallnadel und einem Katheter bekannt. Ein mittlerer Abschnitt der Nadel ist an seiner Außenseite mit einer Isolierung versehen, und ein distales Ende liegt frei. Die Nadel wird beispielsweise bis in die Nähe eines Nervens in den Hals eines Patienten eingeführt. Die Position der Nadelspitze wird durch elektrische Stimulation überprüft. Der Katheter wird dann durch die Nadel eingeführt. An einem Katheteradapter ist eine Metallzunge mit einem spiralförmig gewundenen Draht des Katheters verbunden, so daß ein elektrischer Stimulator mit einem am vorderen Ende des Katheters frelliegenden Draht des Katheters verbunden werden kann. Nach Positionierung des Katheters wird ein Anästhetikum durch den Katheter appliziert, beispielsweise zur Vorbereitung einer Schulteroperation.

Aus der EP 1 181 947 A2 ist ein Epiduralkatheter mit mindestens drei in einer Reihe angeordneten Elektroden bekannt. Die Elektroden dienen zur elektrischen Stimulation von Nerven oder des Rückenmarks. Es kann ein Kanal zur Verabreichung von Medikamenten vorgesehen sein, so daß zusätzlich zur elektrischen Stimulation des Rückenmarks oder der Spinalnerven eine Injektion schmerzstillender Medikamente erfolgen kann.

Ein weiterer implantierbarer Epiduralkatheter ist aus der DE 203 08 422 U1 bekannt. Dieser Katheter ist beispielsweise zur Applikation von gepulster Hochfrequenz zur Nervenstimulation geeignet. Außerdem kann eine Spritze oder eine Medikamentenpumpe angeschlossen werden. Der Katheter weist ein Befestigungselement auf, das zur Befestigung des Katheters an einer Eintrittsstelle in einem Körper dienen kann und durch welches elektrische Zuleitungen und eine Schlauchleitung des Katheters hindurchgeführt sind.

Aufgabe der vorliegenden Erfindung ist es, ein Elektrodensystem der eingangs genannten Art mit einem Katheter oder einer Elektrode zu schaffen, das flexibler einsetzbar ist.

Diese Aufgabe wird erfindungsgemäß gelöst durch eine implantierbare, flexible Elektrode, insbesondere Epiduralelektrode, mit mindestens einem distalen elektrischen Kontakt, wobei die Elektrode einen subkutan implantierbaren Port aufweist, wobei in dem Port mindestens ein mit dem distalen Kontakt verbundenes elektrisches Kontaktelement zur Herstellung einer elektrischen Verbindung mit einem Sondenkontakt einer in den Port einführbaren Sonde angeordnet ist, wobei ein Innenraum des Ports gegenüber der Umgebung durch ein Septum abgeschlossen ist, über welches bei mitsamt dem Port vollständig unterhalb der Haut implantierter Elektrode der Port von extern mittels einer Nadel zugänglich ist, die die Sonde bildet oder aus der die Sonde vorschiebbar ist.

Weiter wird die Aufgabe gemäß Patentanspruch 2 gelöst durch ein Elektrodensystem mit einer implantierbaren, flexiblen Elektrode, insbesondere Epiduralelektrode, mit mindestens einem distalen elektrischen Kontakt, wobei die Elektrode einen subkutan implantierbaren Port aufweist, eine in den Port einführbare Sonde mit mindestens einem Sondenkontakt ein Teil des Elektrodensystems ist, in dem Port mindestens ein mit dem distalen Kontakt verbundenes elektrisches Kontaktelement zur Herstellung einer elektrischen Verbindung mit dem Sondenkontakt angeordnet ist, und ein Innenraum des Ports gegenüber der Umgebung durch ein Septum abgeschlossen ist, über welches bei mitsamt dem Port vollständig unterhalb der Haut implantierter Elektrode der Port von extern mittels einer Nadel zugänglich ist, die die Sonde bildet oder aus der die Sonde vorschiebbar ist. Dabei ist gemäß einer Weiterentwicklung die Elektrode in einem implantierbaren, flexiblen Katheter, insbesondere Epiduralkatheter, angeordnet. In diesem Falle ist in dem Port eine Zugangsöffnung des Katheters angeordnet.

Nach dem Anlegen der Elektrode bzw. des Katheters kann dieser vollständig mitsamt dem Port nahe der Eintrittsstelle in den Körper unterhalb der Haut implantiert werden. Der unter der Haut verborgene Katheter ist für den Patienten weniger hinderlich. Außerdem ist das Infektionsrisiko und die Gefahr von Komplikationen verringert. Das erfindungsgemäße Elektrodensystem erlaubt es daher beispielsweise, nach einer ambulanten Stimulationsbehandlung eines Patienten die Elektrode oder den Katheter im Körper des Patienten zu belassen und den Patienten nach Hause zu entlassen, bis beispielsweise nach einigen Tagen oder Wochen eine erneute Stimulation notwendig wird. Es kann dann beispielsweise eine Sonde in Form einer Nadel in den Port eingeführt werden, um erneut einen Stimulationsstrom zu applizieren. Das erfindungsgemäße Elektrodensystem hat also in der Anwendung deutliche Vorteile gegenüber einer herkömmlichen implantierbaren Elektrode.

In einer besonders bevorzugten Ausführungsform sind das elektrische Kontaktelement und der Sondenkontakt zur Übertragung von Radiofrequenz ausgebildet. Somit kann eine Stimulation mittels Hochfrequenzpulsen stattfinden.

Das Elektrodensystem mit dem Katheter stellt eine Weiterentwicklung dar, die ferner auch gegenüber einem herkömmlichen implantierbaren Katheter oder Stimulationskatheter einen vergrößerten Einsatzbereich erschließt. Wird der Katheter beispielsweise als Epiduralkatheter in den Bereich des Wirbelkanals eingelegt, so kann neben der Stimulation des Rückenmarks oder der Spinalnerven durch beispielsweise einen gepulsten Hochfrequenzstrom nach Bedarf zusätzlich die Injektion eines schmerzstillenden Medikaments durch den Port und die Zugangsöffnung des Katheters erfolgen. Der implantierbare Port kann beispielsweise ein Septum aufweisen, das von extern mit einer Injektionsnadel erreichbar ist.

Wahlweise kann zwischen dem Port und dem Katheter auch eine ebenfalls implantierbare Medikamentenpumpe vorgesehen sein. Dadurch ist eine gleichmäßige Verteilung der Medikamentengabe über einen längeren Zeitraum erreichbar.

Wahlweise ist an dem Port eine Zuspritzkammer für den Katheter angeordnet. Dadurch wird das Zuspritzen von Medikamenten in den Port erleichtert, und die Zuspritzkammer kann außerdem als Vorratskammer für eine implantierbare Medikamentenpumpe dienen.

Weitere vorteilhafte Ausgestaltungen der Erfindung ergeben sich aus den Unteransprüchen.

Vorzugsweise weist die Sonde ein Kupplungselement auf, und der Port der Elektrode oder des Katheters weist eine Ankuppelvorrichtung für das Kupplungselement auf. Dies stellt beispielsweise ein Mittel zur mechanischen Verankerung der Sonde an dem Port dar. Dabei kann es sich beispielsweise zum eine mechanische Halterung oder Verriegelung handeln, beispielsweise einen Klickverschluß. Durch die Ankuppelvorrichtung und das Kupplungselement kann insbesondere ein sicherer Kontakt zwischen dem Sondenkontakt und dem elektrischen Kontaktelement hergestellt werden. Wenn die Sonde an dem Port gehalten wird, wird außerdem beispielsweise das Applizieren von Pulsen zur Nervenstimulation erleichtert.

Vorzugsweise wird das Kupplungselement der Sonde durch einen Gewindeansatz gebildet. Vorzugsweise wird die Ankuppelvorrichtung durch ein Gewindeelement gebildet. In dieses kann beispielsweise die Sonde mit dem Gewindeansatz eingeschraubt werden.

Bei der Elektrode oder dem Katheter weist der Port ein Septum zum Durchstechen mit einer Nadel auf. Beispielsweise wird die Sonde durch die Nadel gebildet. Alternativ nimmt die Nadel die Sonde auf, und die Sonde ist aus der Nadel vorschiebbar. Dabei ist das elektrische Kontaktelement innerhalb des Ports so angeordnet, daß eine elektrische Verbindung zu mindestens einem Sondenkontakt der Sonde herstellbar ist. Außerdem ist beispielsweise im Falle der Ankuppelvorrichtung auch die Ankuppelvorrichtung so in bezug auf das Septum angeordnet, daß die Sonde an die Ankuppelvorrichtung ankoppelbar ist. Ein Septum hat den Vorteil, daß der Innenraum des Ports sowohl mit als auch ohne eingestochene Nadel gegenüber der Umgebung isoliert und abgeschlossen ist.

Vorzugsweise ist ein Führungselement am Port vorgesehen, welches die Sonde zum elektrischen Kontaktelement und gegebenenfalls zur Ankuppelvorrichtung führt.

Im folgenden werden bevorzugte Ausführungsbeispiele anhand der Zeichnung näher erläutert.

Es zeigen:
- Figur 1: eine schematische Darstellung eines Epiduralkatheters mit einem implantierbaren Port sowie einer Nadel, die eine Sonde aufnimmt;
- Figur 2: eine schematische Darstellung eines Längsschnitts durch die Spitze des Epiduralkatheters; und
- Figur 3: eine schematische perspektivische Ansicht des Ports mit eingeführter Sonde.

Aus Gründen der Übersichtlichkeit sind die Zeichnungen nicht maßstabsgetreu.

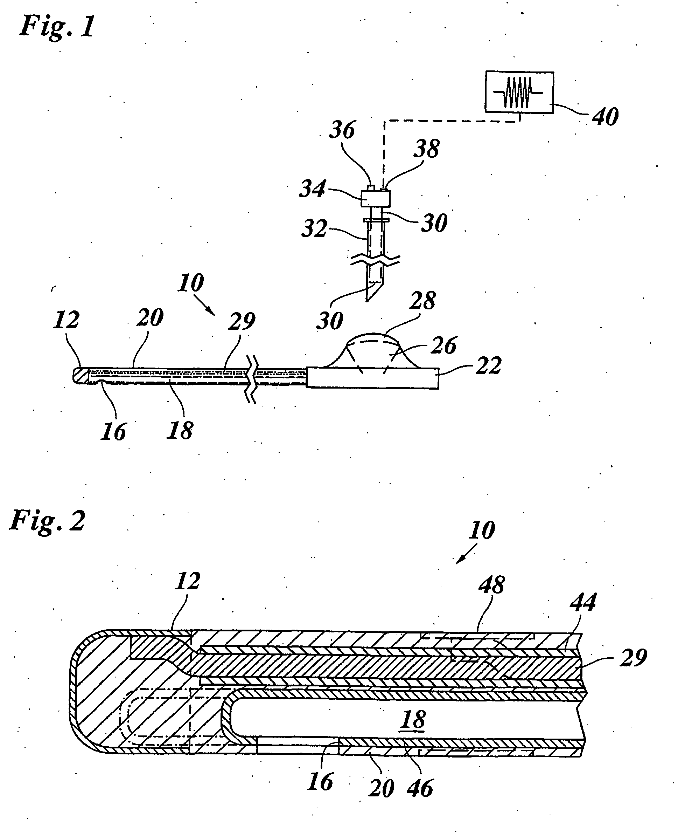

Der in Figur 1 gezeigte Epiduralkatheter 10 weist im distalen Bereich einen elektrischen Kontakt 12 auf, der eine Kappe bildet, die das Ende des Katheters 10 umhüllt. Der Kontakt 12 ist schraffiert dargestellt. Neben dem Kontakt 12 ist eine seitliche Öffnung 16 einer Schlauchleitung 18 des katheters angeordnet. Die Kante des elektrischen Kontakts 12 schließt bündig mit einer aus Silikonkautschuk gefertigten Hülle 20 des Katheters 10 ab. Der Außendurchmesser der Hülle 20 beträgt 1,33 mm entsprechend einer Maßangabe von 4 French. In Längsrichtung des Katheters 10 hat der Kontakt 12 eine Ausdehnung, die in etwa dem Außendurchmesser der Hülle 20 entspricht.

Am proximalen Ende ist der Katheter 10 nahtlos mit einem flachen Gehäuse 22 verbunden. Die obere Wand des Gehäuses 22 weist eine Ausbuchtung auf, in der ein Port 26 ausgebildet ist, dessen obere Wandung durch ein Anstechseptum 28 gebildet wird. Über das Anstechseptum 28 ist der Port 26 von extern beispielsweise mittels einer Injektionsnadel zugänglich. Das Anstechseptum 28 ist in bekannter Weise so gefertigt, daß seine Wand eine hinreichende Dichte und Elastizität aufweist, um sich nach dem Herausziehen einer zuvor hindurchgesteckten Injektionsnadel wieder zuverlässig zu verschließen.

Im Inneren des Katheters 10 verläüft eine elektrische Zuleitung 29 für den elektrischen Kontakt 12 parallel zu der Schlauchleitung 18 in der Hülle 20 und ist wie diese gestrichelt angedeutet. Der Aufbau des Katheters 10 wird weiter unten anhand der Figur 2 näher erläutert.

In bekannter Weise ist in der Schlauchleitung ein nicht dargestellter steriler Führungsdraht angeordnet, der zum Verschieben des Katheters 10 an die gewünschte Position im Wirbelkanal dient und danach zurückgezogen wird. Eine Einführungsöffnung für den Führungsdraht wird vor dem Implantieren des Gehäuses 22 verschlossen. Der Führungsdraht ist im Bereich seines vorderen Endes leicht biegsam.

Die in Figur 1 dargestellte Sonde 30 ist in einer Nadel 32 aufgenommen und verschieblich darin geführt. An ihrem hinteren Ende ist sie mit einem Adapter 34 verbunden, an der ein Anschluß 36 für eine Spritze oder eine Medikamentenpumpe und ein elektrischer Anschluß 38 angeordnet sind. Der elektrische Anschluß 38 ist direkt oder über einen nicht gezeigten Adapter zum Anschluß an einen Pulsgenerator 40 geeignet, der einen gepulsten Hochfrequenzstrom erzeugt. Bei dem Pulsgenerator 40 kann es sich beispielsweise um das Gerät N50 der Firma Stryker How Medica, das Gerät RFG-3C+ der Firma Radionics oder das Gerät Neurotherm der Firma RDG Medical handeln.

Figur 2 zeigt die Spitze des Katheters 10 in einer Darstellung als Längsschnitt. Parallel zur Schlauchleitung 18 verläuft die elektrische Zuleitung 29, die an den elektrischen Kontakt 12 innen angelötet ist. Die elektrische Zuleitung 29 weist eine Isolierung 44 auf und verläuft innerhalb eines verdickten Bereiches der Wand der Hülle 20. Die Schlauchleitung 18 wird durch eine zusätzliche innere, schlauchförmige Schicht 46 in der Hülle 20 gebildet. Die Hülle 20 umschließt dabei sowohl den von der inneren Schicht 46 gebildeten Schlauch als auch die Isolierung 44. Die innere Schicht 46 ist durch die Hülle 20 von dem Kontakt 12 isoliert. Mindestens an der Öffnung 16, die die Schicht 46 und die Hülle 20 durchstößt, ist die innere Schicht 46 dicht mit der Hülle 20 verbunden. Sie kann jedoch auch Teil einer aus zwei oder mehreren Schichten aufgebauten Hülle des Katheters sein.

Der von der inneren Schicht 46 geformte Schlauch endet jenseits der Öffnung 16. Er kann jedoch auch, wie durch strichpunktierte Linien angedeutet ist, bis in die von dem distalen Kontakt 12 gebildete Kappe hineinragen.

Alternativ oder zusätzlich zu dem kappenförmigen elektrischen Kontakt 12 kann jedoch auch ein gestrichelt angedeuteter ringförmiger Kontakt 48 in der Nähe der seitlichen Öffnung 16 vorgesehen sein. Dabei kann alternativ zur seitlichen Öffnung 16 eine Öffnung am Ende des Katheters 10 vorgesehen sein.

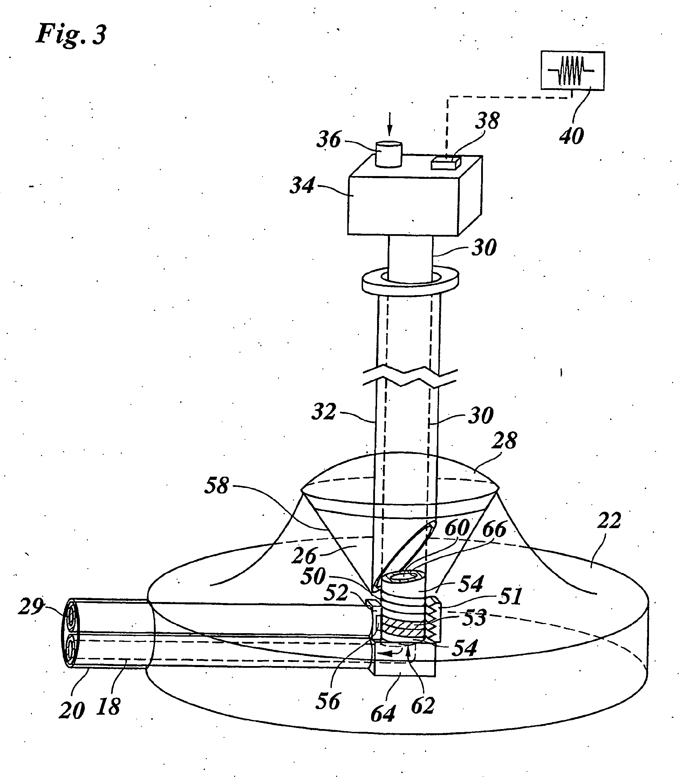

In Figur 3 ist die Nadel 32 durch das Anstechseptum 28 hindurchgestochen, und die Sonde 30 wurde durch die Nadel 32 in den Port 26 eingeführt. Die Sonde 30 ist im unteren Bereich aufgeschnitten dargestellt.

Die Sonde 30 weist an ihrem unteren Ende einen Gewindeansatz 50 auf, der in eine im Gehäuse 22 ausgebildete Fassung mit einem Gewindeelement 51 und einem Gegenstück 52 eingeschraubt ist

Im Bereich des Gewindeansatzes 50 weist die Sonde 30 einen elektrisch leitfähigen Bereich auf, der einen schraffiert dargestellten Sondenkontakt 53 bildet und sich beispielsweise über eine Höhe von zwei Gewindegängen erstreckt. Oberhalb und unterhalb des Sondenkontakts weist die Außenwand der Sonde einen Isolationsmantel 54 auf.

Der Gewindeansatz 50 mit dem Sondenkontakt 53 ist zwischen dem Gewindeelement 51 und dem Gegenstück 52 gehalten, welches ein elektrisches Kontaktelement 56 aufweist. Das elektrische Kontaktelement 56 erstreckt sich über einen begrenzten Höhenbereich des Gegenstücks 52 und liegt etwa auf der Höhe, an der sich der Sondenkontakt 53 befindet, wenn die Sonde 30 bis zu einem Anschlag in die Fassung eingeschraubt ist. Auf diese Weise ist eine elektrische Verbindung zwischen dem Sondenkontakt 53 und dem mit der elektrischen Zuleitung 29 verbundenen elektrischen Kontaktelement 56 hergestellt. Zugleich ist die Sonde 30 mechanisch an das Gehäuse 22 des Ports 26 angekoppelt. Dabei bildet der Gewindeansatz 50 ein Kupplungselement der Sonde 30, und die Fassung mit dem Gewindeelement 51 und dem Gegenstück 52 bildet eine Ankuppelvorrichtung des Ports 26.

Am Port 26 ist unterhalb des Anstechseptums 28 ein Konus 58 angeordnet, der ein Führungselement für die Sonde 30 bildet und so das Plazieren des Gewindeansatzes 50 in dem Port 26 erleichtert. Durch Drehen der Sonde 30 in der Nadel 32 wird dann der Gewindeansatz 50 in die Fassung mit dem Gewindeelement 51 eingeschraubt.

Der Sondenkontakt 53 ist mit dem elektrischen Anschluß 38 des Adapters 34 verbunden. Auf diese Weise wird eine elektrische Verbindung zwischen dem Pulsgenerator 40 und dem elektrischen Kontakt 12 des Katheters 10 hergestellt. Der Kontakt 12, die Zuleitung 29, das elektrische Kontaktelement 56, der Sondenkontakt 53 und die Sonde 30 mit dem Adapter 34 sind sowohl zur Applikation von Pulsen zu einer Teststimulation von Nerven oder des Rückenmarks geeignet, die beispielsweise eine Spannung im Bereich von 0 - 12 V mit einer Frequenz im Bereich von 50 bis 150 Hz und einer Pulsweite im Bereich von 150 bis 400 Mikrosekunden aufweisen können, als auch zu Applikation von gepulster Hochfrequenz mit einer Spannung beispielsweise im Bereich von 20 bis 30 V und einer gepulsten Frequenz von beispielsweise 50 kHz oder 500 kHz mit einer Pulsweite von 20 Millisekunden. Die angegebenen Zahlenwerte sind lediglich Beispiele zur Verdeutlichung der Einsatzbreite des Katheters.

So kann beispielsweise mittels Hochfrequenzpulsen bei einem Epiduralkatheter durch eine Reizung der Nerven innerhalb des Wirbelkanals in vielen Fällen eine Behandlung oder Reizung von Nervengeflechten vor der Wirbelsäule oder in gefährlichen Bereichen mit Spezialnadeln vermieden werden, und es können auch Nerven mit Hochfrequenzpulsen behandelt werden, die ansonsten dafür nicht zugänglich wären.

Im Inneren der Sonde 30 verläuft ein Zugangskanal 60, der am unteren Ende geöffnet ist und am oberen Ende mit dem Anschluß 36 des Adapters 34 verbunden ist. Das untere Ende des Zugangskanals 60 ist bei mit dem Gewindeelement 51 verschraubtem Gewindeansatz 50 vor einer Zugangsöffnung 62 eines Verbindungselements 64 des Ports 26 angeordnet. Über das Verbindungselement 64 ist die Zugangsöffnung 62 mit der Schlauchleitung 18 des Katheters 10 und somit mit der Öffnung 16 verbunden.

Die Wand des Zugangskanals 60 weist eine Isolationsschicht 66 auf, die am unteren Ende der Sonde 30 mit dem Isolationsmantel 54 verbunden ist, so daß der Zugangskanal 60 gegenüber dem Sondenkontakt 53 und dessen Zuleitung zum elektrischen Anschluß 38 elektrisch isoliert ist. Die Sonde 30 ist im gezeigten Beispiel so weit in die Fassung mit dem Gewindeelement 51 eingeschraubt, daß sie dicht an das Verbindungselement 64 abschließt. Dadurch sind der Sondenkontakt 53 und das elektrische Kontaktelement 56 gegenüber dem Zugangskanal 60 und der Zugangsöffnung 62 des Katheters 10 abgedichtet, so daß eine sich im Zugangskanal 60 und der Schlauchleitung 18 befindende Flüssigkeit nicht in Kontakt zu dem Sondenkontakt 53 gerät. Wahlweise kann die Abdichtung beispielsweise auch am Außenumfang des Gewindeansatzes 50 unterhalb des elektrischen Kontaktelements 56 erfolgen.

Die Sonde 30 und der Katheter 10 erlauben es, nach der Implantation des katheters 10 bei einer späteren, weiteren Behandlung des Patienten mit einem Einführen der Nadel 32 mit der Sonde 30 in den Port 26 auszukommen, ohne daß weitere Operationen notwendig wären. Außerdem hat die Nähe des Kontaktes 12 zu der distalen Öffnung 16 des Katheters 10 den Vorteil, daß während des Anlegens des Katheters 10 die Lage des Katheters durch eine Teststimulation mit geringerer Spannung und Frequenz überprüft werden kann. Ein weiterer Vorteil ist, daß der Kontakt einen ausreichenden Röntgenkontrast bietet, um eine Positionierung des Katheters mit Röntgenkontrolle ohne Kontrastmittelgabe zu ermöglichen. Ein Vorteil ist außerdem, daß derselbe Bereich sowohl mit Medikamenten als auch mit elektrischer Stimulation behandelt werden kann, ohne daß die Lage des Katheters verändert werden muß. Über den Anschluß 36 kann somit beispielsweise ein Medikament in den Katheter 10 eingespritzt werden.

Wahlweise kann beispielsweise auf eine Abdichtung der Verbindung zwischen dem Zugangskanal 60 und der Schlauchleitung 18 durch das Verbindungselement 64 oder durch die Fassung mit dem Gewindeelement 51 auch verzichtet werden, da das Innere des Ports 26 durch das Anstechseptum 28 jedenfalls stets gegenüber dem Körper des Patienten abgedichtet ist und ein Kontakt der Flüssigkeit zum elektrischen Kontaktelement 56 gegebenenfalls hinnehmbar ist.

Aus dem beschriebenen Katheter 10 erhält man durch Weglassen der Schlauchleitung 18, der seitlichen Öffnung 16 und des Verbindungselements 64 eine Ausführungsform einer erfindungsgemäßen Epiduralelektrode. Eine daran angepaßte Ausführungsform der Sonde ergibt sich beispielsweise durch Weglassen des Zugangskanals 60 und des Anschlusses 36 an dem Adapter 34.

Die beschriebenen Ausführungsformen der Elektrode, des Katheters und der Sonden sollen eine mögliche Anordnung und Kontaktierung des Sondenkontakts 53 und des elektrischen Kontaktelements 56 sowie eine mögliche Verbindung zwischen einem Zugangskanal 60 und der Schlauchleitung 18 veranschaulichen und eine Möglichkeit der Ausführung des Ports aufzeigen.

Selbstverständlich kann das Elektrodensystem gemäß der Erfindung auch einen hiervon abweichenden Aufbau aufweisen. So kann beispielsweise neben dem elektrischen Kontakt 12 mindestens ein weiterer elektrischer Kontakt vorgesehen sein, und die elektrischen Kontakte können über getrennte Kontaktelemente mit getrennten Sondenkontakten verbunden sein, die beispielsweise auf unterschiedlichen Höhen angeordnet sind. Dabei kann das Gegenstück 52 entsprechend verlängert sein. Weitere elektrische Verbindungen können beispielsweise für einen im distalen Bereich des Katheters angeordneten Temperatursensor vorgesehen sein, dessen Anschlußleitungen im Katheter angeordnet sind.

Auch die Ausführung des Kupplungselements der Sonde 30 und der Ankuppelvorrichtung des Katheters 10 als Gewindeansatz 50 und Gewindeelement 51 mit Gegenstück 52 ist lediglich ein mögliches Ausführungsbeispiel. So ist beispielsweise auch ein einklickbarer Verschluß oder eine sonstige mechanische Verankerung denkbar.

## Patentansprüche

1. Implantierbare, flexible Elektrode, insbesondere Epiduralelektrode, mit mindestens einem distalen elektrischen Kontakt (12),
wobei die Elektrode einen subkutan implantierbaren Port (26) aufweist,
wobei in dem Port (26) mindestens ein mit dem distalen Kontakt (12) verbundenes elektrisches Kontaktelement (56) zur Herstellung einer elektrischen Verbindung mit einem Sondenkontakt (53) einer in den Port (26) einführbaren Sonde (30) angeordnet ist,
**dadurch gekennzeichnet, daß**
ein Innenraum des Ports (26) gegenüber der Umgebung durch ein Septum (28) abgeschlossen ist, über welches bei mitsamt dem Port vollständig unterhalb der Haut implantierter Elektrode der Port (26) von extern mittels einer Nadel (32) zugänglich ist, die die Sonde bildet oder aus der die Sonde (30) vorschiebbar ist.

2. Elektrodensystem mit einer Elektrode nach Anspruch 1 und mit einer in den Port (26) einführbaren Sonde (30) mit mindestens einem Sondenkontakt (53),
wobei das mindestens eine elektrische Kontaktelement (56) zur Herstellung einer elektrischen Verbindung mit dem Sondenkontakt (53) eingerichtet ist,
weiter **dadurch gekennzeichnet, daß**
über das Septum (28) bei mitsamt dem Port (26) vollständig unterhalb der Haut implantierter Elektrode der Port (26) von extern mittels einer Nadel (32) zugänglich ist, die die Sonde bildet oder aus der die Sonde (30) vorschiebbar ist.

3. Elektrodensystem nach Anspruch 2, **dadurch gekennzeichnet, daß** das elektrische Kontaktelement (56) und der Sondenkontakt (56) zur Übertragung von Radiofrequenz ausgebildet sind.

4. Elektrodensystem nach Anspruch 2 oder 3, **dadurch gekennzeichnet, daß** die Sonde (30) ein Kupplungselement (50) aufweist und der Port (26) eine Ankuppelvorrichtung (51, 52) für das Kupplungselement (50) aufweist.

5. Elektrodensystem nach Anspruch 4, **dadurch gekennzeichnet, daß** die Ankuppelvorrichtung (51, 52) ein Gewindeelement (51) aufweist und das Kupplungselement (50) der Sonde (30) einen Gewindeansatz (50) aufweist.

6. Elektrodensystem nach einem der Ansprüche 2 bis 5, **dadurch gekennzeichnet, daß** ein Teil einer Wandung des Ports (26) durch das Septum (28) zum Durchstechen mit der Nadel (32) gebildet wird.

7. Elektrodensystem nach Anspruch 6, **dadurch gekennzeichnet, daß** die Nadel (32) die Sonde (30) aufnimmt und die Sonde (30) aus der Nadel (32) vorschiebbar ist.

8. Elektrodensystem nach einem Ansprüche 2 bis 7, **dadurch gekennzeichnet, daß** die Elektrode in einem implantierbaren, flexiblen Katheter (10), insbesondere Epiduralkatheter (10), angeordnet ist und daß in dem Port (26) eine Zugangsöffnung (62) des Katheters (10) angeordnet ist.

9. Elektrodensystem nach Anspruch 8, **dadurch gekennzeichnet, daß** die Sonde (30) einen Zugangskanal (60) für die Zugangsöffnung (62) des Katheters (10) aufweist.

10. Elektrodensystem nach Anspruch 9, **dadurch gekennzeichnet, daß** der Sondenkontakt (53) gegenüber dem Inneren des Zugangskanals (60) elektrisch isoliert ist.

11. Elektrodensystem nach einem der Ansprüche 9 und 10, **dadurch gekennzeichnet, daß** die Sonde (30) und der Port (26) so gestaltet sind, daß der Sondenkontakt (53) und das elektrische Kontaktelement (56) gegenüber dem Zugangskanal (60) und der Zugangsöffnung (62) des Katheters (10) abdichtbar sind.

12. Implantierbarer, flexibler Katheter, insbesondere Epiduralkatheter, mit einer in dem Katheter angeordneten Elektrode nach Anspruch 1, wobei in dem Port (26) eine Zugangsöffnung (62) des Katheters (10) angeordnet ist.

## Claims

1. Implantable, flexible electrode, particularly an epidural electrode, comprising at least one distal electrical contact (12),
wherein the electrode comprises a subcutaneously implantable port (26),
wherein in the port (26), there is arranged at least one electrical contact element (56) for establishing an electrical connection to a probe contact (53) of a probe (30), which is introduceable into the port (26), the at least one electrical contact element (56) being connected to the distal contact (12),
**characterized in that**
an interior space of the port (26) is sealed against the surroundings by a septum (28), through which the port (26) is accessible from external by a needle (32), when the electrode is be completely implanted, including the port, beneath the skin, the needle (32) forming the probe or the probe (30) being adapted to be pushed forward out of the needle (32).

2. Electrode system comprising an electrode according to claim 1 and comprising a probe (30), which is introduceable into the port (26) and which comprises at least one probe contact (53),
wherein the at least one electrical contact element (56) is adapted for establishing an electrical connection to the probe contact (53),
further **characterized in that**
the port (26) is accessible from external through the septum (28) by a needle (32), when the electrode is be completely implanted, including the port, beneath the skin, the needle (32) forming the probe, or the probe (30) being adapted to be pushed forward out of the needle (32).

3. Electrode system according to claim 2, **characterized in that** the electrical contact element (56) and the probe contact (56) are adapted to transmit radio frequency.

4. Electrode system according to claim 2 or 3, **characterized in that** the probe (30) comprises a coupling member (50), and the port (26) comprises a coupling device (51, 52) for the coupling member (50).

5. Electrode system according to claim 4, **characterized in that** the coupling device (51, 52) comprises a threaded member (51), and the coupling member (50) of the probe (30) comprises a threaded shoulder (50).

6. Electrode system according to one of claims 2 to 5, **characterized in that** a part of a wall of the port (26) is formed by the septum (28) for being punctured by the needle (32).

7. Electrode system according to claim 6, **characterized in that** the needle (32) accommodates the probe (30), and that the probe (30) is adapted to be pushed forward out of the needle (32).

8. Electrode system according to any one claims 2 to 7, **characterized in that** the electrode is arranged in an implantable, flexible catheter (10), particularly an epidural catheter (10), and that an access opening (62) of the catheter (10) is arranged in the port (26).

9. Electrode system according to claim 8, **characterized in that** the probe (30) comprises an access duct (60) for the access opening (62) of the catheter (10).

10. Electrode system according to claim 9, **characterized in that** the probe contact (53) is electrically insulated against the inside of the access duct (60).

11. Electrode system according to any one of claims 9 and 10, **characterized in that** the probe (30) and the port (26) are configured such that the probe contact (53) and the electrical contact element (56) are sealable against the access duct (60) and against the access opening (62) of the catheter (10).

12. Implantable, flexible catheter, particularly an epidural catheter, comprising an electrode according to claim 1, which is arranged in the catheter, wherein an access opening (62) of the catheter (10) is arranged in the port (26).

## Revendications

1. Électrode flexible, implantable, en particulier électrode épidurale, pourvue d'au moins un contact électrique distal (12),
dans laquelle l'électrode comporte un orifice implantable de manière sous-cutanée (26),
dans laquelle au moins un élément de contact électrique (56) relié au contact distal (12) est agencé dans l'orifice (26) pour établir une connexion électrique avec un contact de sonde (53) d'une sonde (30) pouvant être introduite dans l'orifice (26),
**caractérisée en ce que**
un espace intérieur de l'orifice (26) est isolé de l'environnement par un septum (28), par l'intermédiaire duquel lorsque l'électrode est entièrement implantée sous la peau avec l'orifice, l'orifice (26) est accessible depuis l'extérieur au moyen d'une aiguille (32) qui forme la sonde ou à partir de laquelle la sonde (30) peut être avancée.

2. Système d'électrode pourvu d'une électrode selon la revendication 1 et pourvu d'une sonde (30) pouvant être introduite dans l'orifice (26) incluant au moins un contact de sonde (53),
dans lequel le au moins un élément de contact électrique (56) est agencé pour établir une connexion électrique avec le contact de sonde (53),
**caractérisé en outre en ce que**,
lorsque l'électrode implantée est entièrement sous la peau avec l'orifice (26), l'orifice (26) est accessible depuis l'extérieur, par l'intermédiaire du septum (28), au moyen d'une aiguille (32) qui forme la sonde ou à partir de laquelle la sonde (30) peut être avancée.

3. Système d'électrode selon la revendication 2, **caractérisé en ce que** l'élément de contact électrique (56) et le contact de sonde (56) sont conçus pour une transmission par radiofréquence.

4. Système d'électrode selon la revendication 2 ou 3, **caractérisé en ce que** la sonde (30) comporte un élément de couplage (50) et l'orifice (26) comporte un dispositif de couplage (51, 52) pour l'élément de couplage (50).

5. Système d'électrode selon la revendication 4, **caractérisé en ce que** le dispositif de couplage (51, 52) comporte un élément fileté (51) et l'élément de couplage (50) de la sonde (30) comporte un embout fileté (50).

6. Système d'électrode selon l'une des revendications 2 à 5, **caractérisé en ce qu'**une partie d'une paroi de l'orifice (26) est formée par le septum (28) pour être percée avec l'aiguille (32).

7. Système d'électrode selon la revendication 6, **caractérisé en ce que** l'aiguille (32) reçoit la sonde (30) et la sonde (30) peut être avancée à partir de l'aiguille (32)

8. Système d'électrode selon l'une des revendications 2 à 7, **caractérisé en ce que** l'électrode est agencée dans un cathéter flexible, implantable (10), en particulier un cathéter épidural (10), et **en ce qu'**une ouverture d'accès (62) du cathéter (10) est agencée dans l'orifice (26).

9. Système d'électrode selon la revendication 8, **caractérisé en ce que** la sonde (30) comporte un canal d'accès (60) pour l'ouverture d'accès (62) du cathéter (10) .

10. Système d'électrode selon la revendication 9, **caractérisé en ce que** le contact de sonde (53) est isolé électriquement par rapport à l'intérieur du canal d'accès (60).

11. Système d'électrode selon l'une des revendications 9 et 10, **caractérisé en ce que** la sonde (30) et l'orifice (26) sont configurés de telle sorte que le contact électrique (53) et l'élément de contact électrique (56) peuvent être étanchéifiés par rapport au canal d'accès (60) et à l'ouverture d'accès (62) du cathéter (10).

12. Cathéter flexible, implantable, en particulier cathéter épidural, pourvu d'une électrode selon la revendication 1 agencée dans le cathéter, dans lequel une ouverture d'accès (62) du cathéter (10) est agencée dans l'orifice (26).
